# EUROPEAN PATENT SPECIFICATION

(11) **EP 3 629 017 B1**
(45) Date of publication and mention of the grant of the patent: **30.08.2023**
(21) Application number: 19198979.7
(22) Date of filing: 23.09.2019
(51) Int. Cl.: G01N 33/48, G01N 1/04, G01N 1/28, B01L 3/00, A61B 10/00

(54) **PICKUP-SAFE STOOL COLLECTION TUBE**
AUFNAHMESICHERE STUHLSAMMELRÖHRE
TUBE DE COLLECTE DE SELLES SÉCURISÉ POUR LA CAPTURE

(30) Priority: 28.09.2018 DE 102018124132
(43) Date of publication of application: 01.04.2020
(73) Proprietor: Immundiagnostik AG, 64625 Bensheim (DE)
(72) Inventor: ARMBRUSTER, Franz-Paul, 64270 Bobenheim-Roxheim (DE); RUPPERT, Jana, 64625 Bensheim (DE)
(74) Representative: Benedum, Ulrich Max

(56) References cited:
- EP-A1- 1 366 715
- DE-A1-102008 057 866
- DE-U1- 20 321 610
- US-A1- 2004 184 966

## Description

### FIELD OF THE INVENTION

The invention relates to a device for taking specimens of solid fecal material (G01N 33/48, G01N 1/04, A61B 10/02, G01N 1/28) and a tubular device prefilled with a reagent for preservation of a defined amount of stool at ambient temperature and transport to a clinical laboratory for further analysis, in particular, immunochemical testing.

### BACKGROUND OF THE INVENTION

Such a sample device is disclosed in DE 10 2008 057 866 (Gaudlitz GmbH). The sample device, in particular for stool samples, has a container for receiving a liquid reaction and/or solvent, which has an access opening, on which a stripper sleeve is placed in a sealing manner, and a cap member having a sample receiving rod which can be introduced through the stripper sleeve into the container. The cap member can be placed in a sealing manner on the stripper sleeve. The stripper sleeve has a cone-shaped first seal, which is insertable into the access opening of the container under elastic expansion and can be brought into sealing engagement with the inner wall of the container. Additionally or alternatively, the cap member has a cone-shaped second sealing, which can be inserted into an insertion opening of the stripper sleeve under its elastic configuration and can be brought into sealing contact with the inner wall of the stripper sleeve.

The reagent tube consists of a flexible plastic material and is provided with two flanges at the access opening on the outer edge. A sleeve-like intermediate piece is located at the access opening on which two slot hooks are formed laterally for cooperation with a bayonet connection having flanges. The sleeve-like intermediate piece is designed as a cone in the lower section, matching the tube opening, so that after insertion the cone is positioned close-fitting with the inner wall of the tube opening. The inner wall of the intermediate piece narrows in the cone area in the form of a funnel and ends in a funnel opening. At the upper edge of the intermediate piece flanges are formed for a bayonet connection with a lance-like head part, comprising a sample lance, a cover section and a handle. Two bayonet hooks on the outside of the lid section are designed for a bayonet coupling with the flanges of the intermediate piece. In order to engaging the lance-like head part with the sleeve-like intermediate piece, the sample lance is inserted through the funnel opening of the intermediate piece into the reagent tube so that the lower end of the lance carrying the sample comes into contact with the reagent and the analyte is extracted.

In order to avoid leaks during transport of the stool sampling device, the two bayonet couplings between the reagent tube and the intermediate piece, as well as between the intermediate piece and the lance-like head part must be absolutely tight. DE 10 2008 057 866 requires a sealing bead and a sealing shoulder for the sleeve-like intermediate piece on the cone which are so dimensioned that the flexible access opening of the plastic tube is widened by the insertion of the cone. A sealing attachment is also formed in the lance-like head part at the bottom of the cover section by contact with the inner wall of the sleeve-like intermediate piece.

The disadvantage of this system is that reagent solution can leak out from the stool sample device, especially during transport as air freight. Furthermore, the closure system is not practical in the laboratory, since opening and closing the bayonet coupling requires precise handling. Due to the sealing beads and sealing attachments being under pressure, a relatively high application of force and the use both hands is required, which also increases the risk of spillage.

DE 203 21 610 U1 and WO 03/068398 (ScheBo Biotech AG) teach a stool sampling device including a plurality of transverse slots and a reagent tube with a two-piece cap. The intermediate piece comprises a piston-like throttle plate, which is attached to the transverse slots and inserted into the reagent tube. An insertion cone cooperates with the tube wall and an upper sleeve-like section with upper edge slots is provided for bayonet coupling. The inner wall of the intermediate piece is funnel-shaped with a pushthrough opening for the lance-like headboard. The head part comprises a sample lance, a cover section with lateral projections for a twist-plug connection with the slots at the edge of the sleeve, and a handle. For the closure, the sample lance is inserted into the reagent vessel through the opening of the funnel.

The two-piece bayonet fitting is sealed by the accuracy of fit of i) the sample lance and the insertion opening, and ii) the sample lance and insertion opening through the piston-like throttle plate. For operation, the user sticks the sample lance into the stool and then inserts the lance with the sample through the funnel opening into the reagent tube. Thereby, all the sample material protruding beyond the depressions in the lance are stripped off and remain in a hopper. During this process, however, small-sized solid residues such as hard seeds can interfere with the insertion and sealing procedure making the seal between lance rod and insertion opening leaky for the reagent.

The bayonet joint between the lid section and the sleeve edge is also not odour-proof. Another disadvantage is the piston-like throttle plate. The throttle plate must be removed from the tube with two hands, overcoming a vacuum in the tube, similar to such of a plunger syringe - whereby the negative pressure is immediately cleared up. The sudden change in pressure and the inflow of air can cause a spray effect of reagent and stool sample.

EP 1 366 715 (Sentinel CH S. r. L), EP 1384 442 A1 (Alfa Scientific Designs Inc.), JP 06148177 (Fujirebio Inc.), JP 06207935 (Teikoku Seiyaku Co. Ltd), JP10300642 (Dainippon Ink & Chem), JP 10300643 (Dainippon Ink & Chem), JP11316222A (Nissho Corp.), US 5,246,669 (Hayashi et al), US 5,514,341 (Urata et al) teach stool sample devices that are not prefilled and do not extract the analyte in a liquid reagent. DE 196 24 687 A1 (Hamilton Bonaduz AG) teaches a device for taking complicated samples.

DE 10 2012 109 457 teaches an odour-proof stool sampling device. However, the user may easily pull out the sample lance after insertion. This situation may occur, for example, if the user wrongly considers that an insufficient amount of sample has been introduced into the reagent vessel and proceeds to repeat the procedure of sampling collection.

In particular, there is a need of a stool sampling device that can provide simple transferring of a defined quantity of stool into a liquid reagent, wherein leakage of the liquid reagent during transport and storage is prevented. In addition, means for avoidance of repeated sampling collection by the user, leading to subsequent false measurements, is required. Therefore, the state of the art represents a problem.

### SUMMARY OF THE INVENTION

According to the disclosure, the problem is solved by a sampling device with the features of claim 1. Further aspects of the invention are recited in the dependent claims.

The stool sampling device of the disclosure comprises a reagent vessel, an intermediate piece and a sample lance. Said reagent vessel has a top access opening and at least one flange on its outer wall. Said intermediate piece has a cylindrical top opening, at least one integrally formed fitting guide which cooperates with the at least one flange of the reagent vessel, and a bottom plug-in cone. Said intermediate piece has means for close-fitting with the inner wall of the reagent vessel at the access opening, and a bottom fitting hole connecting with the interior of the reagent vessel. Said sample lance has a head part and a lance rod for taking a stool sample, wherein introducing the lance rod into the intermediate piece through the fitting hole results in the closure of the reagent vessel by the head part.

The stool sampling device of the disclosure is **characterized in that** the intermediate piece has a top cylindrical inner wall with at least one sealing bead. The head part of the sample lance has at least one bottom taper sealing ring. The at least one taper sealing ring in the head part of the sample lance cooperates with the least one sealing bead in the intermediate piece, audibly engaging in a snap connection when the lance rod is fully introduced through the fitting hole of the intermediate piece, thereby creating radial tension between the outer wall of the head part and the inner wall of the intermediate piece and radially sealing the access opening of the reagent vessel. At least one fitting recess and at least one fitting window are formed on the cylindrical wall of the intermediate piece. At least one long flange and at least one short ramped flange are integrally formed on the outer wall of the head part. The at least one fitting recess is arranged to receive the at least one long flange, so that the head part is guided into the intermediate piece, thereby allowing the cooperation of the at least one fitting window of the intermediate piece with the at least one short ramped flange and irreversibly restraining the movement of the head part upon engagement with the intermediate piece and closing the reagent vessel.

In one aspect, the stool sampling device may further be characterized in that fitting recesses are formed as essentially opposite pairs on the intermediate piece which are arranged to receive pairs of essentially opposite long flanges integrally formed on the outer wall of the head part.

In another aspect, the stool sampling device may further be characterized in that two essentially opposite fitting windows are formed on the intermediate piece which cooperate with two essentially opposite ramped flanges integrally formed on the outer wall of the head part.

In a further aspect, the stool sampling device may be characterized in that the cylindrical wall of the head part is formed as a hollow cone whose central part is occupied by the lance rod having one or more sample chambers arranged to receive 10 to 15 milligrams stool.

In another aspect, the stool sampling device may be further characterized in that the sealing bead is located at the upper edge of said plug-in cone which has sloped inlet ribs leading to a flat bottom having said fitting hole. Any particles exceeding the stool volume which occupies the sample chambers of the lance rod may be displaced into the gaps between said inlet ribs.

In one aspect, the stool sampling device may be characterized in that a membrane made of an elastic polymer is formed at the bottom part of the plug-in cone, thereby closing the fitting hole.

In another aspect, the stool sampling device may be characterized in that a chamfer is provided at the fitting hole. The chamfer may be arranged to receive a close-fitting rod taper sealing, thereby closing the fitting hole when the lance rod is passed through the plug-in cone.

In a further aspect, the head part the sample lance is provided with a head handle having two essentially opposite head handle ramps arranged to allow application of homogenous pressure on the head part upon insertion of the sample lance through the intermediate piece. Said head handle ramps may be essentially parallel to said short ramped flanges of the intermediate piece.

In one aspect, the stool sampling device may be characterized in that the fitting guide of the intermediate piece has a coupling hook for snapping and holding the at least one vessel flange, wherein the coupling hook and fitting guide are designed to break apart when the intermediate part is turned anti-clockwise around the vessel.

In another aspect, the stool sampling device may be characterized in that said reagent vessel has a bottom grip with two essentially opposite sloped surfaces.

In a further aspect, the stool sampling device may be characterized in that said means for close-fitting with the inner wall of the reagent vessel are in form of least one sealing bead at the access opening of the reagent vessel.

In one preferred aspect, the stool sampling device is provided with a reagent vessel prefilled with a reagent for extraction and stabilization of analytes from stool.

In another aspect, the bottom part of the vessel 12 is provided with an access port which is releasably sealed by a hollow nib that can be easily broken to open the access port and allow dropwise dispensing.

### BRIEF DESORPTION OF THE DRAWINGS

Further aspects and advantages of the stool sampling device of the disclosure are described with reference to the attached drawings. It shows:
- Fig. 1: a spatial view from the side of the intermediate piece in engaged position with the reagent vessel (left), and the sample lance (right);
- Fig. 2: a spatial view from the side of the sample lance in restrained position inserted through the intermediate piece in engaged position with the reagent vessel;
- Fig. 3 A: two spatial views from the side of the head part of the sample lance (left) and from the bottom (right);
- Fig. 3 B: spatial views of the intermediate piece from above (left) and the top part of the reagent vessel (right) in non-engaged position;
- Fig. 4 A: a spatial view from the side of the intermediate piece and the top part of the reagent vessel in engaged position;
- Fig. 4 B: a spatial sectional view of the intermediate piece;
- Fig. 5 A: a spatial view from the side of the head part of the sample lance in restrained position inserted through the intermediate piece in engaged position with the reagent vessel;
- Fig. 5 B: a longitudinal sectional view of the head part of the sample lance inserted through the intermediate piece in engaged position with the reagent vessel according to Fig. 5 A.

### DETAILED DESCRIPTION OF THE INVENTION

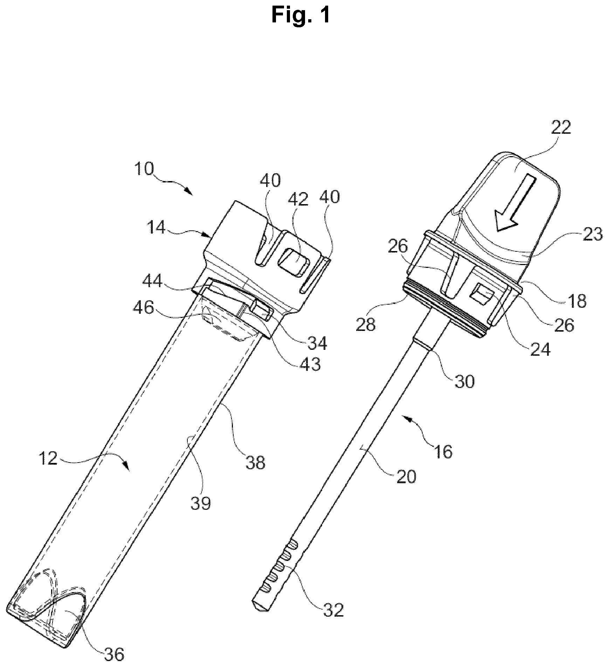

**Fig.1** shows a stool sampling device 10, comprising a reagent vessel 12 and a cylindrical intermediate piece 14 in engaged position (left) and a sample lance 16 (right). The reagent vessel 12 has a cylindrical shape and it is provided with a vessel flange 34 located on the vessel's outer wall 38 at the upper part of the vessel 12, close to the vessel's opening. The bottom part of the intermediate piece 14 is provided with a curved fitting guide 44 comprising a sloped spacing section ending in a coupling hook 43. As shown in Fig. 1 (left), the intermediate part 14 is placed in engaged position at the upper part of the reagent vessel 12, close to its opening. The engagement results from the cooperation between the coupling hook 43 and the vessel flange 34. The engagement may be achieved by positioning the intermediate part 14 on top of the reagent vessel 12, allowing the vessel flange 34 to enter one end of the fitting guide, and turning the intermediate part 14 clockwise around the reagent vessel 12. The vessel flange 34 is then engaged by the coupling hook 43 which is arranged for snapping and holding the vessel flange 34 and, thus, the vessel 12. The intermediate piece 14 is thus stably hold in position by contacting the outer wall 38 of reagent vessel 12 through the cooperation between the coupling hook 43 and the vessel flange 34. The intermediate piece 14 also comprises a plug-in cone 46 which penetrates into the upper part of the vessel 12 contacting the inner wall 39 of the vessel 12. Therefore, the intermediate piece 14 covers up the vessel's opening by contacting both the inner 39 and outer 38 walls of the vessel 12. The bottom part of the reagent vessel 12 is provided with a bottom grip 36 having opposite sloped flat surfaces which allow holding the vessel 12 using two fingers. The flat surfaces of the bottom grip 36 may be connected by a round portion at the very bottom of the vessel 12. This arrangement may be designed to allow stable fitting in a rack of an automatic system for subsequent analysis. The wall 38, 39 of the reagent vessel 12 is designed for a tight radial connection of the plug-in cone 46 with sealing beads (not shown) and it is made of a non-elastic, transparent material such as polypropylene or clear polystyrene.

The intermediate piece 14 may be made of a resilient but, to a certain extent, elastic material such as hard rubber or soft plastic to allow proper sealing when engaged with the vessel 12. The upper part of the intermediated piece 14 is provided with a pair of elongated recesses 40 and a fitting window 42 arranged to receive and hold the head part 18 of the sample lance 16. The sample lance 16 (right) comprises a head part 18 and a lance rod 20 made of resilient plastic material, which are dimensioned to allow cooperation with the vessel 12 and intermediate piece 14 in engaged position. The lance rod 20 may be integrally molded in one piece with the head part 18 and head handle 22. The top part of the head part 18 is provided with a double sided, flat head handle 22 having head handle ramps 23 on both sides which are widening in the direction to the bottom part of the head handle 22. The outer wall 48 of the head part 18 is provided with a short ramped flange 24 and a pair of long flanges 26 which are designed for cooperation with the fitting window 42 and recesses 40 of the intermediate piece 14, respectively. The lower edge of the head part 18 is provided with a taper sealing ring 28 designed for a sealing cooperation with the inner wall of the intermediate piece 14.

The upper part of the lance rod 20 is provided with a rod taper sealing 30 designed for cooperation with the inner wall of the plug-in cone 46. The lance rod 20 is, close to the head part 18 and above the rod taper sealing 30, broader than the section below the rod taper sealing 30, which is narrowing in the direction to the bottom end of the lance rod 20. The lower section of the rod 20 is provided with one or more sample chambers 32 which are depressions designed to capture a defined amount of test material when inserted in a sample of stool. The sample chambers 32 may be located perpendicularly to the axis of the lance rod 20, preferably in opposite pairs. The sample chambers 32 may be provided with internal ribs for improved retention of stool material, while promoting an upwards movement of hard particles in the stool when sliding the lance rod 20 through the fitting hole 58. These undesired particles are then displaced into spaces or gaps provided for this purpose and do not get wedged in the fitting hole 58. A specific amount of stool material is critical for the subsequent analysis so that only the exact amount of material contained in the depressions of the sample chambers 32 must be used for analysis. Particularly preferred are recesses 32 on the lance rod 20 having a volume of at least 12 cubic millimetres, corresponding to at least 10 milligrams stool and a maximum of 15 milligrams stool.

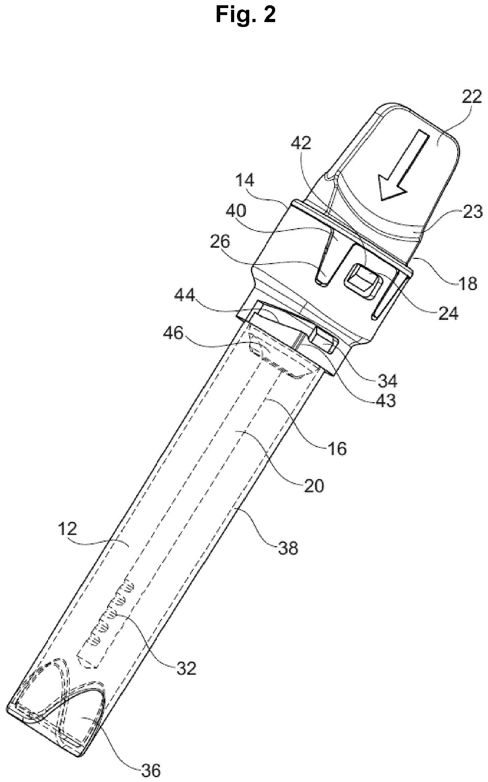

**Fig. 2** shows a stool sampling device 10 comprising a reagent vessel 12 and a cylindrical intermediate piece 14 in engaged position by the cooperation of a coupling hook 43 with a vessel flange 34, and a sample lance 16 inserted through the intermediate piece 14 into the reagent vessel 12. The sample lance 16 is introduced vertically (arrow) through the intermediate piece 14 by positioning the pair of long flanges 26 of the head part 18 in line with the pair of recesses 40 of the intermediate piece 14. The head handle 22 is provided at one end of the head part 18 for holding and positioning of the sample lance 16. Thus, the sample lance 16 can only be inserted in one possible orientation, which is determined by the position of the long flanges 26 in respect to the the recesses 40. Once the insertion position of the sample lance 16 is guided by the recesses 40, the user can pushed the sample lance 16 through the intermediate piece 14, which involves the user applying certain pressure with two fingers on the provided head handle ramps 23. As the head part 18 slides through the intermediate piece 14, the short ramped flange 24 on the head part 18 pushes sideways the wall section of the intermediate part 14 located between the recesses 40 until the short ramped flange 24 reaches the fitting window 42. The ramp of the short flange 24 facilitates the head part 18 sliding along the inner wall of the intermediate piece 14. At this stage, as shown in Fig. 2, the short ramped flange 24 protrudes throughout the intermediate piece 14, occupying the fitting window 42. The shape of flange 24, having a flat upper edge protruding and contacting the upper edge of the fitting window 42, impedes the movement of the head part 18 inserted through the intermediate piece 14 in any direction. In this position, the sample rod 20 has passed through the plug-in cone 46 into the interior of the reagent vessel 12, thereby allowing the material contained in the depressions of the samples chambers 32 to come in contact with a reagent solution (not shown). Such liquid reagent may be used for sample preparation and/or for the extraction of one or more analytes from the stool sample material. One or more sealing beads (not shown) for tight radial connection may be formed on the inner wall of the vessel 12, which provide cooperating means with one or more sealing rings (not shown) on the outer wall of the plug-in cone 46 so that a sealing of the interface between vessel 12 and plug-in cone 46 is provided. When the sample lance 16 is inserted through the plug-in cone 46, the taper sealing ring 28 (Fig. 1) of the head part 18 cooperates with a sealing bead 56 on the inner wall of the intermediate piece 14 for sealing the opening of the reagent vessel 12, which prevents both spillage of liquids and release of unpleasant odours. The bottom part of the vessel 12 may be provided with an access port which is releasably sealed by a hollow nib that can be easily broken to open the access port and allow convenient dropwise dispensing (not shown).

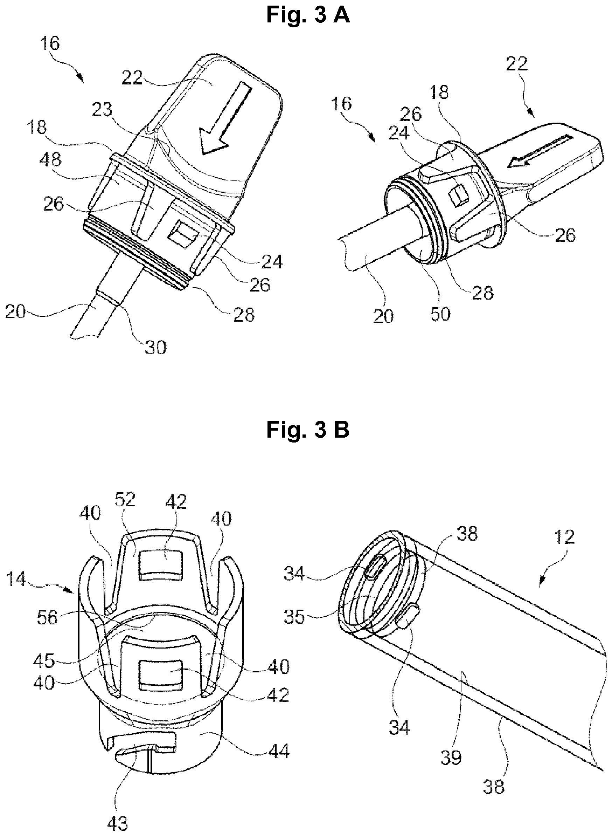

**Fig. 3** **A** shows the head part 18 of the sample lance 16 from two perspective views. The left view shows the head part 18 from the side having a pair of long flanges 26 as elongated protrusions extending from the upper edge of the wall 48 along the main body of the head part 18, whereas the bottom end on the flange 26 does not reach the area of the taper sealing ring 28. Between the long flanges 26, a short ramped flange 24 is formed as a cuboid protrusion extending from the wall 48. The short flange 24 is provided with a ramp section on its bottom half. The taper sealing ring 28 has a smaller circumference than the main body of the head part 18 and can be formed with at least one ring for cooperation with the intermediate piece 14, whereas the central section of the sealing ring 28 may have a greater circumference than the top and bottom sections of the taper sealing ring 28.

Extending from the upper part of the main body of the head part 18, a head handle 22 is provided having head handle ramps 23 on each side of the handle 22 thickening into the base of the handle 22. The orientation of the short 24 and long flanges 26 of the head part 18 is essentially parallel to the head handle 22. The arrow indicates the inserting direction of the sample lance 16 and the position of the fingers of the user to apply pressure on the handle ramps 23, which project into a widening base of the head handle 22 for increased stability. The lance rod 20 is provided with a rod taper sealing 30, which defines the interface between two differentials circumferential dimensions within the lance rod 20, the upper part being wider than the portion below the rod taper sealing 30.

The right perspective view in **Fig. 3** **A** shows the head part 18 from the bottom. The head handle 22 extends from the top part of the main body of the head part 18. The main body of the head part 18 has a cylindrical shape consisting of an outer wall 48 and an inner wall 50 which form a hollow cone whose central part is occupied by the lance rod 20, which also extends from the upper part of the main body of the head part 18.

**Fig. 3** **B** shows on the left drawing a perspective view of the intermediate piece 14 from the top opening 45. The head part 18 is, after insertion of the lance rod 20, to be introduced into the intermediate piece 14 through the top opening 45. Four fitting recesses 40 are formed on the upper part of the intermediate piece 14, which are essentially provided as opposing pairs. A respective fitting window 42 is formed between each pair of fitting recesses 40, which are located essentially opposite to each other. The location of each pair of recesses 40 is required to guide the long flanges 26 of the head part 18 and to allow a certain degree of bending of the wall to accommodate the incoming short flange 24 which is pushing sideways the wall as the head part 18 is inserted through the intermediated piece 14. The short ramped flange 24 has essentially the same base surface area as the dimensions of the fitting window 42. Once the short ramped flange 24 reaches and pops out through the fitting window 42, the wall portion between the long flanges 26 returns to its original position. The inner wall 52 of intermediate piece 14 is provided with a sealing bead 56 to accommodate and engage the taper sealing ring 28 of the head part 18 into a snap connection. The lower end of the recesses 40 does not contact the sealing bead 56. Accordingly, as the head part 18 is introduced into the intermediate piece 14, the sealing ring 28 cooperates with the sealing bead 56 simultaneously to the short ramped flange 24 occupying the fitting window 42, thereby restraining the movement of the head part 18 in any vertical or rotational direction. The bottom part of the intermediate piece 14 is provided with a fitting guide 44 which is designed to engage the reagent vessel 12. The fitting guide 44 is formed as a two part piece having a curved, long sloped spacing section leading to a shorter spacing section in form of a coupling hook 43. In one aspect, two fitting guides 44 are located essentially opposite to each other.

**Fig.** 3 B shows on the right drawing a perspective view of the reagent vessel 12 having two vessel flanges 34 formed on the outer wall 38 close to the top opening 35 of the vessel 12, which are essentially opposite to each other. The vessel flanges 34 are designed for cooperation with the fitting guide 44 and the coupling hook 43.

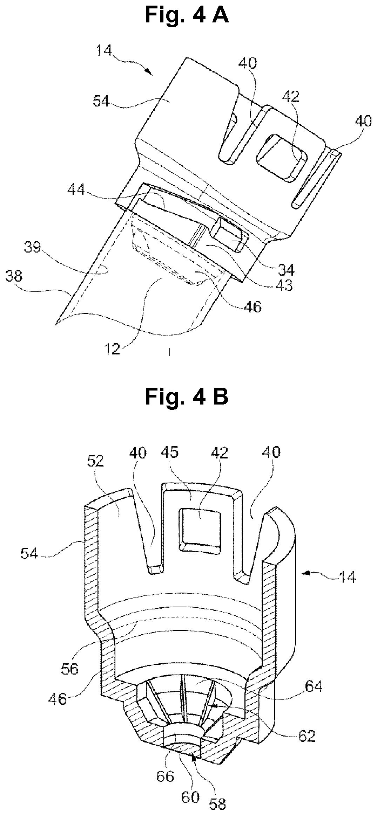

**Fig.** 4 A shows the top part of the vessel 12 in engaged position with the intermediate piece 14 through cooperation of the coupling hook 43 and the vessel flange 34. The intermediate piece 14 covers up the top opening 35 of the vessel 12 embracing the outside wall 38 of the vessel 12 and having a close-fitting interaction through the plug-in cone 46 with the inner wall 39 of the vessel 12. Thereby, the top opening 35 is sealed at the interface between the inner wall 39 of the vessel 12 and plug-in cone of the intermediate piece 14. The fitting guide 44 is so dimensioned that it allows the accommodation and sliding of the flanges 34 when the intermediate piece 14 is placed over the opening of vessel 12. The flanges 34 and coupling hook 43 are preferably arranged in such a way that a clockwise rotation of intermediate piece 14 around the vessel 12 results in an snapping position of the coupling hook 43 over the flanges 34, restraining the movement of the intermediate piece 14. A snapping sound indicates to the operator that the intermediate piece 14 is sealingly held on the upper edge of the vessel 12. The sliding and hooking procedure is easily carried out without the application of excessive force (it is not foreseen to be done by the user). Although the snapping connection can be opened-up by a reverse movement and the intermediate piece 14 thereby released, it requires a certain force to be applied, thereby avoiding an accidental opening of the vessel 12 by the user. Importantly, the coupling hook 43 and fitting guide 44 are designed to break apart when the intermediate part 14 is turned anti-clockwise around the vessel 12, thereby separating the vessel 12 and the intermediate part 14 from each other. Should the user open the vessel 12, for example, to refill the tube with more sample, the broken coupling hook 43/fitting guide 44 would then indicate that the content of the device is not suitable for analysis. The system 10 is designed to allow a single sampling event, which guarantees that the specific amount of sample necessary for the subsequent test has been introduced into the vessel 12. In addition, one or more sealing rings (not shown) can be provided on the outer wall 54 of the intermediate piece 14 at the upper part of the plug-in cone 46 for isolating the interface between the intermediate piece 14 and vessel 12. The reagent vessel 12 may also carry one or more sealing beads on the inner wall 39 at the area contacting a sealing ring of the plug-in cone 46, so that both elements fit sealingly. The reagent vessel 12 preferably consists of a solid, non yielding plastic material, while the plug-in cone 46 and intermediate piece 14 are made of a softer, elastic material for efficient seal.

**Fig. 4B** shows a spatial sectional view depicting the intermediate piece 14 in half. A pair of fitting recesses 40 and a fitting window 42 located between the recesses 40 are formed on the wall (52, 54) at the upper part of the intermediate piece 14, which has a broader circumference than the bottom part corresponding to the plug-in cone 46. The inner wall 52 of the intermediate piece 14 is essentially circular-cylindrical and may have variable thickness in different sections, in particular, it may have an upper section with sufficient diameter to allow the insertion of the head part 18 of the sample lance 16, following into one or more narrowing sections to create radial tension with the inserting head part 18, while promoting the cooperation of the sealing ring 28 with the sealing bead 56.

The inner wall of the vessel 12 close to its opening 35 may cooperate with the outer wall 54 of the plug-in cone 46 at its upper part by means of a sealing bead/ring cooperation type (not shown) so as to seal the interface between the vessel 12 and the plug-in cone 46. A sealing bead 56 is located on the inner wall 52 between the upper part of the intermediate piece 14 and the plug-in cone 46 for cooperation with the sealing ring 28 of the head part 18. The position or depth of the sample lance 16 inside the intermediate piece 14 is determined and limited cooperatively by the fitting window 42 and the sealing bead 28.

An opening 58 for precisely fitting with the lance rod 20 is provided at the bottom part of the plug-in cone 46. The inner bottom part of the plug-in cone 46 does not end in the form of a funnel. Instead, the fitting hole lies in an essentially flat base. The inner part of the plug-in cone starts in a star-shaped guide opening towards the inlet ribs 62, which guide the lance rod 20 into the fitting hole 58, having gaps 64 between the ribs 62 for the accommodation of excess stool and any hard particles. The fitting hole 58 and lance rod 20 stand under tension, so that a seal at the opening 58 is given.

The inner part of the plug-in cone 46 is provided with sloped inlet ribs 62 narrowing as they extend into the bottom part of the plug-in cone 46 thereby forming gaps 64 for collection of particles exceeding the size and amount captured by the sample chambers 32 of the sample lance 16 upon insertion. The inlet ribs 62 lead to a chamfer 66 at the upper edge of the fitting hole 58 that connects the top opening 45 with the interior of the vessel 12. The chamfer 66 is arranged to receive a rod taper sealing 30. When the lance rod 20 is inserted through the intermediate piece 14, the first, thinner section of the rod 20 penetrates into the vessel until the close-fitting cooperation of chamfer 66 and taper sealing 30 provides a further stop, which results in an additional surface for enhanced closure and isolation against odours. The fitting hole 58 has a circumference matching the diameter of the thinner section of the lance rod 20, thereby stripping out excessive stool material and impeding it to enter the vessel 12, which material is accumulated in the gaps 64 between the inlet ribs 62. The fitting hole 58 may be covered with a thin membrane 60 of polymer material for isolating the content, i.e. reagent solution, of the vessel 12 prior insertion of the sample lance 16. This preferably elastic membrane 60 can easily be perforated by the tip of the lance rod 20. The membrane 60 may thus be made of an elastic material, rubber or elastomer. Alternatively, the membrane 60 can be made of cellulose. The membrane 60 allows perforation and aperture of the fitting hole 58 by applying sufficient pressure with the sample lance 16. The membrane 60 represents and additional level of isolation against odours since it intimately surrounds the lance rod 20 due to its elastic property.

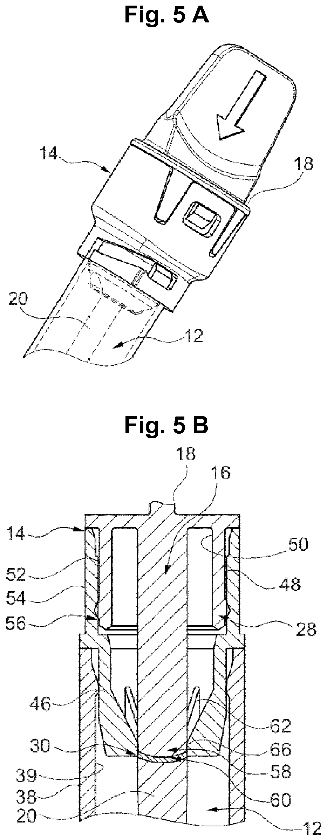

**Fig. 5A** shows a spatial view from the side of the head part 18 of the sample lance 16 in restrained position inserted through the intermediate piece 14 and the reagent vessel 12 in engaged position, whereas the lance rod 20 passes though the plug-in cone 46.

**Fig. 5B** shows a longitudinal sectional view of the head part 18 of the sample lance 16 inserted through the intermediate piece 14 and the reagent vessel 12 in engaged position, whereas the lance rod 20 passes though the plug-in cone 46, according to Fig. 5 A. The sectional view does not include any flanges 24, 26 of the head part 18, or recesses 40 or fitting window 42 of the intermediate piece 14. The inner wall 52 of the intermediate piece 14 interacts with the outer wall 48 of the head part in a close-fitting manner. The sealing ring 28 of the head part 18 cooperates in a snap connection with the sealing bead 56 of the intermediate piece 14 close to the upper edge of the plug-in cone 46, which has a smaller diameter than the upper part of the intermediate piece 14. The wall 48, 50 of the head part 18 forms a hollow cone, wherein the lance rod 20 extends from the upper part of the head part 18 occupying the central part of the cone, thereby creating a space. The lance rod 20 is introduced through the plug-in cone 46 guided by the sloping inlet-ribs 62 reaching the fitting hole 58, which matches the diameter of the section of the lance rod 20 below the taper sealing 30. In this position, there are two independent spaces, above and below the fitting hole 58, isolated from each other. The spacing above the fitting hole 58 is arranged to receive all the excessive stool material and it is permanently kept closed after insertion of the sample lance 16. The space below the fitting hole 58 is contacted by the rod 20 with its section of smaller diameter that carries the sample to be analyzed in the depressions of the sample chambers 32. The vessel 12 may be sealed at the contacting interface with the intermediate piece 14 by the cooperation of a sealing bead located on the outer wall of the plug-in cone 46 and a sealing ring located on the inner wall the vessel 12, or vice versa.

The sealing ring 28 on the head part 18 may be slightly wider than the diameter of a lower portion of the inner wall 52 of the intermediate piece 14 just before the plug-in cone 56, so that radial sealing and tensioning are achieved even before reaching the sealing bead 56. Another radial seal may be provided at the fitting hole 58 which tensions the lance rod 20.

The lance rod 20 may not have the same thickness everywhere. An upper section may be provided with increased cross-section in relation to the lower section carrying the specimen and, in particular, having a cross-section slightly larger than the cross-section of the fitting hole 58. When the sample lance 16 with the sample is passed through the fitting hole 58, the user can apply force to create tension with the upper section of the lance rod 20 against the fitting hole 58. Thereby, the cross-section of the fitting hole 58 increases being radially expanded, which also results in radial expansion of the plug-in cone 46, clamping against the inner wall of the reagent vessel 12 with a higher clamping force.

Not shown is the stool sample device 10 in the opening position after insertion and engaging of the sample lance 16, for collecting a sample of the reagent solution with extracted analytes from stool for subsequent analysis, without any risk of contamination by stool material comprised within the hollow cone of the head part 18 and the intermediate piece 14. By releasing the tension between the vessel flange 34 and the coupling hook 43, the intermediate piece 14 can be rotated anti-clockwise, while the sample lance 16 remaining engaged, thereby sliding the vessel flange 34 along the fitting guide 44 to its end. Then, the sample lance 16 attached to the intermediate piece 14 can be pulled out from the vessel 12. The cooperation of the flanges 24 with the fitting window 42 as well as the snap connection of the sealing ring 28 with the sealing bead 56 provide a strong attachment of sample lance 16 with the intermediate piece 14 so that no accidental disengagement, and spillage of stool material, can occur.

For use, the operator takes the sample lance 16 and inserts it several times into different areas of a sample of feaces so that the depressions or chambers 32 at the end of the lance rod 20 are filled with stool. Next, the user holds the vessel 12 engaged to the intermediate piece 14 by the bottom grip 36 of the vessel 12 in one hand. There is no risk of spillage or leak if the fitting hole 58 of the intermediate piece 14 is sealed with an elastic membrane 60. With the other hand, the user holds the sample lance 16 at the head handle 22 and introduces the tip of the lance rod 20 vertically into the top opening 45 of the intermediate piece 14.

When the lance rod 20 is introduced, it is vertically guided along the inlet ribs 62, leading to the chamfer 66 into the fitting hole 58. The user must then apply certain pressure on the head handle ramps 23 to pierce the membrane 60, thereby introducing the lance rod 20 through the fitting hole 58 into the vessel 12. Sample material protruding from the recesses or chambers 32 or stool material adhering to the wall of the rod 20 is stripped off, pressed into the chambers 32 and/or shifted into the gaps 64 between the inlet ribs 62.

As the user stabs the lance rod 16 into the intermediate piece 14 and the head part 18 approximates the intermediate piece 14, the user must place the long flanges 26 of the head part 18 in line with the recesses 40 of the intermediate piece 14 to be able to continue inserting the sample lance 16. As the head part 18 is guided in depth by the recesses 40, the short ramped flange 24 slightly pushes sideways the wall 52, 54 between the recesses 40 as the ramp contacts the wall. When the long flanges 26 contact the bottom part of the recesses 40 and the short flange 24 reaches the fitting window 42, popping throughout the wall, the head part 18 is restrained and cannot move in any vertical or rotational direction. As the user pushes down the lance 16 to let the flange 24 pop out through the fitting window 42, the sealing ring 28 is pressed against the sealing bead 56 and its cooperation results in a snap connection, closing and sealing the opening vessel 12 and the intermediate piece 14, so that no odours or liquids may escape from the sampling device 10.

The depressions 32 in the sample lance 16 have a predetermined volume. The stool sample is thus introduced into the reagent and the analyte already extracted. This eliminates the risk of further contamination. The intermediate piece 14 lock does not have to be opened by the operator. The intermediate piece 14 engaged with the sample lance 16 is only opened at arrival in the analytical laboratory.

The stool sampling device of the disclosure provides odour-proof sealing by means of the at least one taper sealing ring in the head part of the sample lance cooperating with the least one sealing bead in the intermediate piece. This sealing is advantageous for providing a homogenous radial tension and tight interaction but also for providing the user with a audible signal indicating proper engagement in a snap connection when the lance rod is fully introduced through the fitting hole of the intermediate piece. In addition, the dimensions and material properties of intermediate piece and head part promote that also radial tension between the outer wall of the head part and the inner wall of the intermediate piece is created, further increasing the radial sealing. Furthermore, the chamfer arranged to receive a close-fitting rod taper sealing represents an additional sealing, reducing even more the risk of odour leakage.

The sampling of stool performed by patients themselves is commonly associated with the wrong assumption that the material collected in the chambers 23 might be insufficient to perform the subsequent test. If the patient repeats the sampling procedure, the quantity of sample introduced in the reagent vessel would be translated in a higher concentration of analyte in the vessel. The result would be a false positive and, thus, a wrong diagnosis. The prior art sampling devices do not consider this aspect of stool sampling and therefore they do not address this problem. The sampling device of the disclosure provides means for avoidance of false positives and wrong diagnosis, which requires the repetition of tests, thereby delaying the diagnosis process and increasing the medical costs. The problem is solved by the provision of restraining means. As the head part is guided by the recesses, the short ramped flange protrudes at the fitting window, resulting in a sample lance being blocked, without the possibility to be removed from the vessel. This approach does not require the use of excessive force and it is highly effective in avoiding repetition of stool sampling.

The prior art teach systems that are under high pressure to provide efficient sealing. However, this approach leads to increased risk of pillage when the sampling device is to be opened at arrival in the clinical laboratory, for example, due to uncontrolled use of force to release the cover part. This may also lead to the formation of a vacuum inside the tube that may result is the sudden clearance of negative pressure upon opening. The sudden change in pressure and the inflow of air can cause a spray effect of the reagent and the stool sample. The sampling device of the disclosure provides a fitting guide with a coupling hook for snapping and holding the vessel at the vessel flange. This approach facilitates the opening of the vessel without a direct, sudden release of pressure, by establishing a two-step process. First, the tension of the cooperation at the coupling hook is released, whereas the intermediate piece is stll covering the vessel. Then, rotating and sliding smoothly the intermediate piece from the coupling hook in the contrary direction allows the safe removal of the cover of the vessel.

### LIST OF REFERENCE NUMBERS

- 10: Stool sampling device
- 12: Reagent vessel
- 14: Intermediate piece
- 16: Sample lance
- 18: Head part
- 20: Lance rod
- 22: Head handle
- 23: Head handle ramp
- 24: Short ramped flange
- 26: Long flange
- 28: Taper sealing ring
- 30: Rod taper sealing
- 32: Sample chambers
- 34: Vessel flange
- 35: Vessel opening
- 36: Bottom grip
- 38: Vessel outer wall
- 39: Vessel inner wall
- 40: Fitting recess
- 42: Fitting window
- 43: Coupling hook
- 44: Fitting guide
- 45: Top opening
- 46: Plug-in cone
- 48: Head outer wall
- 50: Head inner wall
- 52: Intermediate piece inner wall
- 54: Intermediate piece outer wall
- 56: Sealing bead
- 58: Fitting hole
- 60: Sealing membrane
- 62: Inlet rib
- 64: Gap between inlet ribs
- 66: Chamfer

## Claims

1. A stool sampling device (10) comprising a reagent vessel (12), an intermediate piece (14) and a sample lance (16),
said reagent vessel (12) having a top access opening (35) and at least one flange (34) on its outer wall (38);
said intermediate piece (14) having a cylindrical top opening (45), at least one integrally formed fitting guide (24) which cooperates with the at least one flange (34) of the reagent vessel (12), and a bottom plug-in cone (46), said intermediate piece (14) having means for close-fitting with the inner wall (39) of the reagent vessel (12) at the access opening (35), and a bottom fitting hole (58) connecting with the interior of the reagent vessel (12); and
said sample lance (16) having a head part (18) and a lance rod (20) for taking a stool sample,
wherein introducing the lance rod (20) into the intermediate piece (14) through the fitting hole (58) results in the closure of the reagent vessel (12) by the head part (18);
**characterized in that**
the intermediate piece (14) has a top cylindrical inner wall (52) with at least one sealing bead (56);
the head part (18) of the sample lance (16) has at least one bottom taper sealing ring (28);
the at least one taper sealing ring (28) in the head part (18) of the sample lance (16) cooperates with the least one sealing bead (56) in the intermediate piece (14), audibly engaging in a snap connection when the lance rod (20) is fully introduced through the fitting hole (58) of the intermediate piece (14), thereby creating radial tension between the outer wall (48) of the head part (18) and the inner wall (52) of the intermediate piece and radially sealing the access opening (35) of the reagent vessel (12);
at least one fitting recess (40) and at least one fitting window (42) are formed on the cylindrical wall (52, 54) of the intermediate piece (14), and at least one long flange (26) and at least one short ramped flange (24) are integrally formed on the outer wall (48) of the head part (18);
wherein the at least one fitting recess (40) is arranged to receive the at least one long flange (26), so that the head part (18) is guided into the intermediate piece (14), thereby allowing the cooperation of the at least one fitting window (42) of the intermediate piece (14) with the at least one short ramped flange (24) and irreversibly restraining the movement of the head part (18) upon engagement with the intermediate piece (14) and closing the reagent vessel (12).

2. Stool sampling device according to any claim 1, **characterized in that** fitting recesses (40) are formed as essentially opposite pairs on the intermediate piece (14) which are arranged to receive pairs of essentially opposite long flanges (24) integrally formed on the outer wall (48) of the head part (18).

3. Stool sampling device according to any claim 1 or 2, **characterized in that** two essentially opposite fitting windows (42) are formed on the intermediate piece (14) which cooperate with two essentially opposite ramped flanges (26) integrally formed on the outer wall (48) of the head part (18).

4. Stool sampling device according to any one of claims 1 to 3, **characterized in that** the cylindrical wall (48, 50) of the head part (18) is formed as a hollow cone whose central part is occupied by the lance rod (20) having one or more sample chambers (32) arranged to receive 10 to 15 milligrams stool.

5. Stool sampling device according to any one of claims 1 to 4, **characterized in that** the sealing bead (56) is located at the upper edge of said plug-in cone (46) having sloped inlet ribs (62) leading to a flat bottom having said fitting hole (58), configured such that any particles exceeding the stool volume which occupies the sample chambers (32) of the lance rod (20) are displaced into the gaps (64) between said inlet ribs (62).

6. Stool sampling device according to any one of claims 1 to 5, **characterized in that** a membrane made of an elastic polymer (60) is formed at the bottom part of the plug-in cone (46), thereby closing the fitting hole (58).

7. Stool sampling device according to any one of claims 1 to 6, **characterized in that** a chamfer (66) is provided at the fitting hole (58) which chamfer (66) is arranged to receive a close-fitting rod taper sealing (30), thereby closing the fitting hole (58) when the lance rod (20) is passed through the plug-in cone (46).

8. Stool sampling device according to any one of claims 1 to 7, wherein the head part (18) is provided with a head handle (22) having two essentially opposite head handle ramps (23) arranged to allow application of homogenous pressure on the head part (18) upon insertion of the sample lance (16) into the intermediate piece (14), wherein said head handle ramps (23) are essentially parallel to said short ramped flanges (24) of the intermediate piece (14).

9. Stool sampling device according to any one of claims 1 to 8, **characterized in that** the fitting guide (44) of the intermediate piece (14) has a coupling hook (43) for snapping and holding the at least one vessel flange (34), wherein the coupling hook 43 and fitting guide 44 are designed to break apart when the intermediate part 14 is turned anti-clockwise around the vessel 12.

10. Stool sampling device according to any one of claims 1 to 9, **characterized in that** said reagent vessel (12) has a bottom grip (36) with two essentially opposite sloped flat surfaces.

11. Stool sampling device according to any one of claims 1 to 9, **characterized in that** said means for close-fitting with the inner wall (39) of the reagent vessel (12) are in form of least one sealing bead at the access opening (35).

12. Stool sampling device according to any one of the claims 1 to 11, wherein the reagent vessel (12) is prefilled with a reagent for extraction and stabilization of analytes from stool.

13. Stool sampling device according to any one of the claims 1 to 12, wherein the bottom part of the vessel 12 is provided with an access port which is releasably sealed by a hollow nib that can be easily broken to open the access port and allow dropwise dispensing.

## Patentansprüche

1. Stuhlprobenaufnahmevorrichtung (10) mit einem Reagenziengefäß (12), einem Zwischenstück (14) und einer Probenaufnahmelanze (16),
wobei das Reagenziengefäß (12) eine obere Zugangsöffnung (35) und mindestens einen Flansch (34) an seiner Außenwand (38) aufweist;
das Zwischenstück (14) eine zylindrische obere Öffnung (45), mindestens eine einstückig ausgebildete Passführung (24), die mit dem mindestens einen Flansch (34) des Reagenziengefäßes (12) zusammenwirkt, und einen unteren Einsteckkonus (46) aufweist, wobei das Zwischenstück (14) Mittel zur engen Passung mit der Innenwand (39) des Reagenziengefäßes (12) an der Zugangsöffnung (35) und ein unteres Passloch (58) aufweist, das mit dem Inneren des Reagenziengefäßes (12) verbunden ist; und
wobei die Probenlanze (16) ein Kopfteil (18) und einen Lanzenstab (20) zur Entnahme einer Stuhlprobe aufweist,
wobei das Einführen des Lanzenstabes (20) in das Zwischenstück (14) durch das Passloch (58) zum Verschließen des Reagenziengefäßes (12) durch das Kopfteil (18) führt;
**dadurch gekennzeichnet, dass**
das Zwischenstück (14) eine obere zylindrische Innenwand (52) mit mindestens einem Dichtwulst (56) aufweist;
das Kopfteil (18) der Probenaufnahmelanze (16) mindestens einen unteren Kegeldichtring (28) aufweist;
der mindestens eine Kegeldichtring (28) im Kopfteil (18) der Probenaufnahmelanze (16) mit dem mindestens einen Dichtungswulst (56) im Zwischenstück (14) zusammenwirkt, wobei er beim vollständigen Einführen des Lanzenstabes (20) durch das Passloch (58) des Zwischenstücks (14) hörbar in eine Schnappverbindung eingreift, wodurch eine radiale Spannung zwischen der Außenwand (48) des Kopfteils (18) und der Innenwand (52) des Zwischenstücks entsteht und die Zugangsöffnung (35) des Reagenziengefäßes (12) radial abdichtet;
dass an der zylindrischen Wand (52, 54) des Zwischenstücks (14) mindestens eine Einpassaussparung (40) und mindestens ein Einpassfenster (42) ausgebildet sind, und dass an der Außenwand (48) des Kopfteils (18) mindestens ein langer Flansch (26) und mindestens ein kurzer Rampenflansch (24) einstückig ausgebildet sind;
wobei die mindestens eine Einpassaussparung (40) so angeordnet ist, dass sie den mindestens einen langen Flansch (26) aufnimmt, so dass das Kopfteil (18) in das Zwischenstück (14) hineingeführt wird, wodurch das Zusammenwirken des mindestens einen Einpassfensters (42) des Zwischenstücks (14) mit dem mindestens einen kurzen Rampenflansch (24) ermöglicht wird und die Bewegung des Kopfteils (18) beim Eingriff in das Zwischenstück (14) irreversibel zurückgehalten wird und das Reagenziengefäß (12) verschlossen wird.

2. Stuhlprobenaufnahmevorrichtung nach Anspruch 1, **dadurch gekennzeichnet, dass** auf dem Zwischenstück (14) im Wesentlichen gegenüberliegende Paare von Einpassausnehmungen (40) ausgebildet sind, die ausgebildet sind zur Aufnahme von Paaren von im Wesentlichen gegenüberliegenden langen Flanschen (24), die auf der Außenwand (48) des Kopfteils (18) einstückig angeformt sind.

3. Stuhlprobenaufnahmevorrichtung nach einem der Ansprüche 1 oder 2, **dadurch gekennzeichnet, dass** an dem Zwischenstück (14) zwei im Wesentlichen gegenüberliegende Passfenster (42) ausgebildet sind, die mit zwei im Wesentlichen gegenüberliegenden, an der Außenwand (48) des Kopfteils (18) einstückig ausgebildeten Rampenflanschen (26) zusammenwirken.

4. Stuhlprobenaufnahmevorrichtung nach einem der Ansprüche 1 bis 3, **dadurch gekennzeichnet, dass** die zylindrische Wand (48, 50) des Kopfteils (18) als Hohlkegel ausgebildet ist, in dessen Mittelteil die Lanzenstab (20) mit einer oder mehreren Probenkammern (32) zur Aufnahme von 10 bis 15 Milligramm Stuhl angeordnet ist.

5. Stuhlprobenaufnahmevorrichtung nach einem der Ansprüche 1 bis 4, **dadurch gekennzeichnet, dass** sich der Dichtungswulst (56) am oberen Rand des Einsteckkonus (46) befindet, der schräge Einlassrippen (62) aufweist, die zu einem flachen Boden führen, der die Einpassöffnung (58) aufweist, wobei alle Partikel, die das Stuhlvolumen überschreiten, das die Probenkammern (32) des Lanzenstabs (20) einnimmt, in die Lücken (64) zwischen den Einlassrippen (62) verdrängt werden.

6. Stuhlprobenaufnahmevorrichtung nach einem der Ansprüche 1 bis 5, **dadurch gekennzeichnet, dass** am unteren Teil des Einsteckkonus (46) eine Membran aus einem elastischen Polymer (60) ausgebildet ist, die das Einsteckloch (58) verschließt.

7. Stuhlprobenaufnahmevorrichtung nach einem der Ansprüche 1 bis 6, **dadurch gekennzeichnet, dass** eine Abschrägung (66) an der Einpassöffnung (58) vorgesehen ist, wobei die Abschrägung (66) so angeordnet ist, dass sie eine enganliegende Stangenkonusdichtung (30) aufnimmt, wodurch die Einpassöffnung (58) verschlossen wird, wenn die Lanzenstange (20) durch den Einsteckkonus (46) geführt wird.

8. Stuhlprobenaufnahmevorrichtung nach einem der Ansprüche 1 bis 7, wobei das Kopfteil (18) mit einem Kopfgriff (22) versehen ist, der zwei im Wesentlichen gegenüberliegende Kopfgrifframpen (23) aufweist, die so angeordnet sind, dass sie beim Einführen der Probenaufnahmelanze (16) in das Zwischenstück (14) einen gleichmäßigen Druck auf das Kopfteil (18) ausüben, wobei die Kopfgrifframpen (23) im Wesentlichen parallel zu den kurzen Rampenflanschen (24) des Zwischenstücks (14) verlaufen.

9. Stuhlprobenaufnahmevorrichtung nach einem der Ansprüche 1 bis 8, **dadurch gekennzeichnet, dass** die Passführung (44) des Zwischenstücks (14) einen Kupplungshaken (43) zum Einschnappen und Halten des mindestens einen Gefäßflansches (34) aufweist, wobei Kupplungshaken (43) und Passführung (44) so ausgebildet sind, dass sie auseinanderbrechen, wenn das Zwischenstück (14) gegen den Uhrzeigersinn um das Gefäß gedreht wird.

10. Stuhlprobenaufnahmevorrichtung nach einem der Ansprüche 1 bis 9, **dadurch gekennzeichnet, dass** das Reagenziengefäß (12) einen Bodengriff (36) mit zwei im Wesentlichen gegenüberliegenden schrägen Flächen aufweist.

11. Stuhlprobenaufnahmevorrichtung nach einem der Ansprüche 1 bis 9, **dadurch gekennzeichnet, dass** die Mittel für den dichten Abschluss mit der Innenwand (39) des Reagenziengefäßes (12) in Form von mindestens einem Dichtungswulst an der Zugangsöffnung (35) ausgebildet sind.

12. Stuhlprobenaufnahmevorrichtung nach einem der Ansprüche 1 bis 11, wobei das Reagenziengefäß (12) mit einem Reagenz zur Extraktion und Stabilisierung von Analyten aus dem Stuhl vorgefüllt ist.

13. Stuhlprobenaufnahmevorrichtung nach einem der Ansprüche 1 bis 12, wobei der untere Teil des Gefäßes (12) mit einer Zugangsöffnung versehen ist, die lösbar durch eine hohle Feder verschlossen ist, die mühelos aufgebrochen werden kann, um die Zugangsöffnung zu öffnen und eine tropfenweise Abgabe zu ermöglichen.

## Revendications

1. Dispositif de prélèvement de selles (10) comprenant un récipient de réactifs (12), une pièce intermédiaire (14) et une lance de prélèvement d'échantillons (16),
le récipient de réactifs (12) ayant une ouverture d'accès supérieure (35) et au moins une bride (34) sur sa paroi extérieure (38) ;
la pièce intermédiaire (14) présente une ouverture supérieure cylindrique (45), au moins un guide d'ajustement (24) formé d'une seul pièce, qui coopère avec la au moins une bride (34) du récipient de réactifs (12), et un cône d'insertion inférieur (46), la pièce intermédiaire (14) comprenant des moyens pour s'ajuster étroitement avec la paroi intérieure (39) du récipient de réactifs (12) au niveau de l'ouverture d'accès (35) et un trou d'ajustement inférieur (58) communiquant avec l'intérieur du récipient de réactifs (12) ; et
dans lequel la lance de prélèvement (16) comprend une partie de tête (18) et une tige de lance (20) pour prélever un échantillon de selles,
dans lequel l'insertion de la tige de lance (20) dans la pièce intermédiaire (14) à travers le trou d'ajustement (58) entraîne la fermeture du récipient de réactifs (12) par la partie de tête (18) ;
**caractérisé en ce que**
la pièce intermédiaire (14) présente une paroi intérieure cylindrique supérieure (52) avec au moins un bourrelet d'étanchéité (56) ;
la partie de tête (18) de la lance de réception d'échantillons (16) présente au moins une bague d'étanchéité conique inférieure (28) ;
l'au moins une bague d'étanchéité conique (28) dans la partie de tête (18) de la lance de réception d'échantillon (16) coopère avec l'au moins un bourrelet d'étanchéité (56) dans la pièce intermédiaire (14),
dans lequel il s'engage de manière audible dans une liaison par encliquetage lors de l'introduction complète de la tige de lance (20) à travers le trou d'ajustage (58) de la pièce intermédiaire (14),
ce qui crée une tension radiale entre la paroi extérieure (48) de la partie de tête (18) et la paroi intérieure (52) de la pièce intermédiaire et rend l'ouverture d'accès (35) du récipient de réactif (12) radialement étanche ;
**en ce que** sur la paroi cylindrique (52, 54) de la pièce intermédiaire (14) sont formés au moins un évidement d'ajustement (40) et au moins une fenêtre d'ajustement (42), et **en ce que** sur la paroi extérieure (48) de la partie de tête (18) sont formées d'un seul tenant au moins une longue bride (26) et au moins une courte bride de rampe (24) ;
l'au moins un évidement d'ajustement (40) étant disposé de manière à recevoir l'au moins une bride longue (26),
de sorte que la partie de tête (18) est insérée dans la pièce intermédiaire (14),
permettant ainsi la coopération de la au moins une fenêtre d'ajustement (42) de la pièce intermédiaire (14) avec la au moins une bride de rampe courte (24) et retenant de manière irréversible le mouvement de la partie de tête (18) lors de son engagement dans la pièce intermédiaire (14) et fermant le récipient de réactif (12).

2. Dispositif de prélèvement de selles selon la revendication 1, **caractérisé en ce que** des paires sensiblement opposées d'évidements d'ajustement (40) sont formées sur la pièce intermédiaire (14) et sont adaptées pour recevoir des paires de longues brides sensiblement opposées (24) formées d'un seul tenant sur la paroi extérieure (48) de la partie de tête (18).

3. Dispositif de prélèvement de selles selon l'une des revendications 1 ou 2, **caractérisé en ce que** deux fenêtres d'ajustement (42) sensiblement opposées sont formées sur la pièce intermédiaire (14) et coopèrent avec deux brides de rampe (26) sensiblement opposées et formées d'un seul tenant sur la paroi extérieure (48) de la partie de tête (18).

4. Dispositif de prélèvement de selles selon l'une quelconque des revendications 1 à 3, **caractérisé en ce que** la paroi cylindrique (48, 50) de la partie de tête (18) est formée comme un cône creux dans la partie centrale duquel est disposée la tige de lance (20) avec une ou plusieurs chambres de prélèvement (32) destinées à recevoir 10 à 15 milligrammes de selles.

5. Dispositif de prélèvement d'échantillons de selles selon l'une quelconque des revendications 1 à 4, **caractérisé en ce que** le bourrelet d'étanchéité (56) est situé au niveau du bord supérieur du cône d'insertion (46), qui comporte des nervures d'entrée inclinées (62) menant à un fond plat comportant le trou d'insertion (58), toutes les particules dépassant le volume de selles occupant les chambres d'échantillons (32) de la tige de lance (20) étant repoussées dans les espaces (64) entre les nervures d'entrée (62).

6. Dispositif de prélèvement d'échantillons de selles selon l'une quelconque des revendications 1 à 5, **caractérisé en ce qu'**une membrane en polymère élastique (60) est formée à la partie inférieure du cône d'emboîtement (4), fermant ainsi le trou d'emboîtement (58).

7. Dispositif de prélèvement d'échantillons de selles selon l'une quelconque des revendications 1 à 6, **caractérisé en ce qu'**un chanfrein (66) est prévu sur le trou d'insertion (58), le chanfrein (66) étant agencé pour recevoir un joint d'étanchéité de cône de tige (30) étroitement ajusté, fermant ainsi le trou d'insertion (58) lorsque la tige de lance (20) est passée à travers le cône d'insertion (46)

8. Dispositif de prélèvement d'échantillons de selles selon l'une quelconque des revendications 1 à 7, dans lequel la partie de tête (18) est munie d'une poignée de tête (22) comprenant deux rampes de poignée de tête (23) sensiblement opposées, agencées de manière à exercer une pression uniforme sur la partie de tête (18) lors de l'insertion de la lance de prélèvement d'échantillons (16) dans la pièce intermédiaire (14), les rampes de poignée de tête (23) étant sensiblement parallèles aux brides de rampe courtes (24) de la pièce intermédiaire (14).

9. Dispositif de prélèvement d'échantillons de selles selon l'une quelconque des revendications 1 à 8, **caractérisé en ce que** le guide d'ajustement (44) de la pièce intermédiaire (14) comprend un crochet d'accouplement (43) pour encliqueter et maintenir ladite au moins une bride de récipient (34), le crochet d'accouplement (43) et le guide d'ajustement (44) étant configurés pour se rompre lorsque la pièce intermédiaire (14) est tournée dans le sens inverse des aiguilles d'une montre autour du récipient.

10. Dispositif de prélèvement de selles selon l'une quelconque des revendications 1 à 9, **caractérisé en ce que** le récipient de réactif (12) comporte une poignée de fond (36) avec deux surfaces inclinées sensiblement opposées, de prélèvement sur tabouret selon l'une quelconque des revendications 1 à 9, **caractérisé en ce que** ledit récipient à réactifs (12) présente une poignée de fond (36) avec deux surfaces inclinées essentiellement opposées.

11. Dispositif de prélèvement d'échantillons de selles selon l'une des revendications 1 à 9, **caractérisé en ce que** les moyens de fermeture étanche avec la paroi interne (39) du récipient à réactifs (12) sont réalisés sous la forme d'au moins un bourrelet d'étanchéité au niveau de l'ouverture d'accès (35).

12. Dispositif de prélèvement d'échantillons de selles selon l'une quelconque des revendications 1 à 11, dans lequel le récipient à réactifs (12) est prérempli avec un réactif pour l'extraction et la stabilisation d'analytes à partir des selles.

13. Dispositif de prélèvement de selles selon l'une des revendications 1 à 12, dans lequel la partie inférieure du récipient (12) est munie d'une ouverture d'accès obturée de manière amovible par un ressort creux qui peut être aisément rompu pour ouvrir l'ouverture d'accès et permettre une distribution goutte à goutte.
